# EUROPÄISCHE PATENTSCHRIFT

(11) **EP 3 360 503 B1**
(45) Veröffentlichungstag und Bekanntmachung des Hinweises auf die Patenterteilung: **08.09.2021**
(21) Anmeldenummer: 17155560.0
(22) Anmeldetag: 10.02.2017
(51) Int. Cl.: A61B 46/00, A61B 46/20, A61B 46/23

(54) **WEGWERFBARE OPERATIONSABDECKUNG**
DISPOSABLE SURGICAL COVER
COUVERTURE CHIRURGICALE JETABLE

(43) Veröffentlichungstag der Anmeldung: 15.08.2018
(73) Patentinhaber: Paul Hartmann AG, 89522 Heidenheim (DE)
(72) Erfinder: Leistenschneider, Denise, 89429 Bachhagel (DE)

(56) Entgegenhaltungen:
- EP-A1- 1 051 124
- DE-A1-102008 029 051
- DE-T2- 69 811 646
- US-A- 4 169 472
- US-A- 4 489 720
- US-A1- 2005 224 081
- US-A1- 2016 206 393
- US-B1- 6 213 124
- US-B2- 6 694 981
- COTTENDEN DAVID J ET AL: "A study of friction mechanisms between a surrogate skin (Lorica soft) and nonwoven fabrics", JOURNAL OF THE MECHANICAL BEHAVIOR OF BIOMEDICAL MATERIALS, Bd. 28, 1. Dezember 2013 (2013-12-01), Seiten 410-426, XP028772223, ISSN: 1751-6161, DOI: 10.1016/J.JMBBM.2013.04.024

## Beschreibung

Die vorliegende Erfindung betrifft eine wegwerfbare Operationsabdeckung mit einer Längsrichtung und mit einer Querrichtung und mit einer Oberseite und einer Unterseite umfassend ein flüssigkeitsundurchlässiges Basistuchelement mit einem ersten und einem zweiten Querrand, und mit einem ersten und einem zweiten Längsrand, und mit einer Längsmittelachse und mit einer Quermittelachse und mit einem einzigen innerhalb des Basistuchelements angeordneten Lochausschnitt. Der Lochausschnitt ist von einem Folienelement überfangen und das Folienelement ist zumindest im Bereich des Lochausschnitts auf der Unterseite klebend ausgebildet, und weist insbesondere eine Klebebeschichtung auf. Weiter weist das Basistuchelement zumindest in einem Randbereich des Lochausschnitts ein transparentes Flachbahnmaterial auf. Ebenso sind ein Flüssigkeitsauffangbeutel und ein absorbierender Materialabschnitt vorgesehen.

Die Ausstattung von wegwerfbaren Operationsabdeckungen mit Komponenten, wie beispielsweise einem klebenden Folienelement im Lochausschnitt, transparenten Flachbahnmaterialien im Randbereich des Lochausschnitts, einem absorbierenden Materialabschnitt oder einem Flüssigkeitsauffangbeutel, sei es einzeln oder in Kombination, ist bekannt.

Folienelemente im Lochausschnitt mit einer klebenden Unterseite ermöglichen ein keimfreies Abdichten des späteren Eingriffsfeldes.

Transparente Bereiche in einer Operationsabdeckung, wie beispielsweise in US 6,694,981 B2 gezeigt, verschaffen eine Einsehbarkeit in die am Eingriffsbereich direkt benachbarte Umgebung.

An eine wegwerfbare Operationsabdeckung können Beutel zum Auffangen von Flüssigkeit angebracht sein, wie beispielsweise WO 90/06731 A1 zeigt.

OP-Abdeckungen mit am Lochausschnitt angeordneten Flüssigkeitsauffangbeuteln mit einer flüssigkeitsdichten Verschlussmöglichkeit und mit einer (optionalen) Ausstattung an Verstärkungsabschnitten um den Lochausschnitt sind in US 6,213,124 B1 gezeigt.

Das Dokument US 4,169,472 zeigt eine OP-Abdeckung, die im Wesentlichen konzipiert ist für Operationen im Bereich des Kopfes/Schädels, mit einem Lochausschnitt, wobei der Lochausschnitt mit einem auf der Unterseite mit Kleber ausgestatteten Folienelement überdeckt ist und auf der Oberseite der OP-Abdeckung sogenannte "flaps", wie beispielsweise als unverklebte aufstehende Randbereiche eines absorbierenden Materialabschnitts, für die Leitung von Flüssigkeit in einen Flüssigkeitsauffangbeutel vorgesehen sind.

Absorbierende Materialabschnitte dienen der Absorption von während des Eingriffs freigesetzten Flüssigkeiten als dass solche Materialabschnitte auch die Möglichkeit einer Zwischenablage von Operationsinstrumenten bieten, wie beispielsweise aus US 3,738,359 bekannt ist.

US 2005/00224081 A1 lehrt eine Augen-OP-Abdeckung mit einem auf der Oberseite der OP-Abdeckung mittig zwischen zwei Lochausschnitten angebrachten Abdeckelement. Mit einer nur in einem Randbereich vorgenommenen Fixierung des Abdeckelements kann das Abdeckelement auf das nicht für die Operation vorgesehene Auge umgelenkt und damit auf dem dieses Auge zu umgebenden Lochausschnitt platziert werden.

Des Weiteren kann die Ausgestaltung einer wegwerfbaren Operationsabdeckung auf deren unterschiedliche Nutzung und Anwendung abgestimmt sein. So kann eine Operationsabdeckung beispielsweise für eine Ablegung oberhalb und auf dem Patienten vorgesehen sein. Alternativ kann die Anwendung einer Operationsabdeckung in Form einer vertikalen, vorhangartigen Aufhängung vorgesehen sein, um das Operationsfeld und den umgebenden sterilen Bereich von einem unsterilen patientenzugewandten Bereich räumlich abzutrennen. Es sind auch hybride Ausgestaltungen möglich.

Ausgehend von dem bekannten Stand der Technik liegt nun der Erfindung die Aufgabe zugrunde, eine komfortable und kostengünstige wegwerfbare Operationsabdeckung mit einer Grundausstattung an Komponenten bereitzustellen, die in der Anwendung als eine oberhalb und auf dem Patienten abzulegende und dabei an einer Längsachse des Patienten orientierte Operationsabdeckung universal einsetzbar ist. Die wegwerfbare Operationsabdeckung sollte insbesondere bezogen auf den weitgehend symmetrischen Aufbau des menschlichen Körpers, der sich vor allem im Skelettaufbau in der Anordnung von linken und rechten Gliedmaßen zeigt, universal eingesetzt werden können.

Diese Aufgabe wird gelöst durch eine wegwerfbare Operationsabdeckung mit den Merkmalen des Anspruchs 1.

Die erfindungsgemäße wegwerfbare Operationsabdeckung zeichnet sich dabei in der Summe ihrer Merkmale im Wesentlichen dadurch aus, dass der Lochausschnitt auf der Quermittelachse angeordnet ist, dass ein Flüssigkeitsauffangbeutel mit einer dem Lochausschnitt zugewandten Beutelöffnung auf der Oberseite des Basistuchelements festgelegt ist, und der Flüssigkeitsauffangbeutel dabei auf der Quermittelachse in der Querrichtung zwischen dem Lochausschnitt und dem ersten oder dem zweiten Längsrand angeordnet ist, und des Weiteren, dass ein absorbierender Materialabschnitt auf der Oberseite des Basistuchelements unlösbar festgelegt ist, wobei der absorbierende Materialabschnitt auf der Quermittelachse in der Querrichtung zwischen dem Lochausschnitt und dem jeweils anderen ersten oder zweiten Längsrand angeordnet ist.

Es wurde erkannt, dass eine derartig in Reihe angelegte Anordnung von Flüssigkeitsauffangbeutel, Lochausschnitt und absorbierendem Materialabschnitt entlang der Quermittelachse positiv zur universellen Einsetzbarkeit der wegwerfbaren Operationsabdeckung beiträgt, da die wegwerfbare Operationsabdeckung keiner festgelegten oben-unten Orientierung und damit Zuordnung unterliegt. Die erfindungsgemäße Operationsabdeckung kann in Längsrichtung entlang der Längsachse des Patienten sowohl mit dem ersten Querrand oder auch mit dem zweiten Querrand in Richtung Kopf aufgelegt werden.

Die erfindungsgemäße Operationsabdeckung kann mit Blick auf die Position des Operationseingriffsbereiches, sei es links oder rechts der Längsachse des Patienten, wie beispielsweise linke Hüfte oder rechte Hüfte, derart um 180° gedreht werden, dass der Lochausschnitt proximal auf der Eingriffsstelle befestigt werden kann, und dabei der absorbierende Materialabschnitt vorteilhaft von der Eingriffsstelle medial zum Patienten angeordnet ist und damit oberhalb des Körpers des Patienten abgelegt werden kann und der Flüssigkeitsauffangbeutel hingegen lateral der Eingriffsstelle angeordnet ist.

Die erfindungsgemäße Operationsabdeckung kann auch entsprechend der Aufstellung des Ärzteteams und der Assistenz innerhalb des Operationssaales, sei es ob der Chirurg links oder rechts von der Längsachse des Patienten steht, bedarfsgerecht gedreht werden, um für den einzelnen Operationsvorgang eine optimale Anordnung der Komponenten zu erlangen.

Mit "Oberseite" wird eine Seite der wegwerfbaren Operationsabdeckung oder einer der Lagen der Operationsabdeckung verstanden, die im Anwendungszustand vom Patienten abgewandt ist.

Mit "Unterseite" wird die andere, im Anwendungszustand dem Patienten zugewandte Seite der wegwerfbaren Operationsabdeckung oder einer der Lagen der Operationsabdeckung verstanden.

Als "Längsrichtung" wird dabei jegliche Erstreckung zwischen zwei sich gegenüber liegenden Querrändern der wegwerfbaren Operationsabdeckung verstanden. Die "Längsrichtung" der wegwerfbaren Operationsabdeckung ist im Anwendungsfall dabei an der Längsachse eines Patienten, so beispielsweise einer seitlichen Liegeposition des Patienten, ausgerichtet. Als "Längserstreckung" wird ein sich in der Längsrichtung erstreckender Bereich verstanden.

Die "Querrichtung" ist die Richtung senkrecht zur Längsrichtung. Entsprechend ist die "Quererstreckung" als die flächige Ausgestaltung eines in Querrichtung verlaufenden Bereichs zu verstehen.

Die Längsrichtung bzw. Querrichtung der Operationsabdeckung wird dem Anwender, also dem medizinischen Personal, vorteilhafterweise durch Orientierungshilfen angezeigt. Als Orientierungshilfen sind dabei verschiedene Ausführungen denkbar, so beispielsweise in Form von Symbolen, Grafiken, Pfeilen mit Richtungsangabe oder auch als schematische Darstellung von Körperteilen wie beispielsweise Gliedmaßen, oder als Piktogramme der Komponenten (Flüssigkeitsauffangbeutel, absorbierender Materialabschnitt), welche dem Anwender die Orientierung der Längsrichtung bzw. Querrichtung der Operationsabdeckung suggerieren.

"Untere Gliedmaßen" werden hierin als der Anteil am menschlichen Skelett verstanden, welcher ausgehend vom unteren Rumpf, insbesondere Bereich des Beckens, umfassend Hüfte sich über Oberschenkel, Knie, Unterschenkel, Fußgelenk bis inklusive in den Bereich des Fußes erstreckt.

"Auf der Quermittelachse" bzw. "auf der Längsmittelachse" versteht sich als die Anordnung einer Komponente innerhalb des Basistuchelements, bei der die Quermittelachse bzw. die Längsmittelachse die Komponente in ihrer flächenhaften Erstreckung zumindest tangiert oder kreuzt.

"Wegwerfbar" bedeutet, dass die Operationsabdeckung zum insbesondere einmaligen Gebrauch bestimmt ist, also sich insbesondere um eine Einmal-Operationsabdeckung handelt. Das heißt eine wegwerfbare Operationsabdeckung ist nicht dafür geeignet und vorgesehen nach Gebrauch gereinigt oder gewaschen und anschließend wiederverwendet zu werden.

Vorteilhafte Weiterbildungen der wegwerfbaren Operationsabdeckung ergeben sich aus den nachfolgend beschriebenen Ausführungsformen und den jeweiligen Unteransprüchen.

In einer bevorzugten Ausführung sind der Lochausschnitt, der absorbierende Materialabschnitt und der Flüssigkeitsauffangbeutel in der Längsrichtung zentriert auf der Quermittelachse angeordnet.

Damit sind diese vorgenannten Komponenten mit ihrer flächenhaften Erstreckung beidseits der Quermittelachse gleichmäßig verteilt, das heißt, damit auch gleich weit vom ersten und zweiten Querrand entfernt, was eine universale Anwendung der wegwerfbaren Operationsabdeckung noch weiter vorteilhaft unterstützt.

Insbesondere ist der Lochausschnitt auf der Längsmittelachse angeordnet ist.

In einer bevorzugten Ausführungsform ist der Lochausschnitt in der Querrichtung zentriert auf der Längsmittelachse angeordnet. Das bedeutet, dass sich der Lochausschnitt in seiner Form beidseits der Längsmittelachse gleichmäßig verteilt, und der Lochausschnitt zum ersten und zweiten Längsrand einen gleichen Abstand aufweist.

Die Anordnung des Lochausschnitts auf der Längsmittelachse, insbesondere in der Querrichtung zentriert auf der Längsmittelachse, trägt ebenso vorteilhaft zur universalen Anwendbarkeit der Operationsabdeckung bei.

In einer bevorzugten alternativen Ausführungsform kann vorgesehen sein, dass der Lochausschnitt in der Querrichtung nicht zentriert auf der Längsmittelachse angeordnet ist, so dass ein Abstand des Lochausschnitts zu dem ersten Längsrand kleiner oder größer ist als ein Abstand des Lochausschnitts zu dem zweiten Längsrand.

Insbesondere bevorzugt kann dabei vorgesehen sein, dass der Lochausschnitt eine Lochausschnittmittellängsachse aufweist und dass die Lochausschnittmittellängsachse in einem Abstand a versetzt zur Längsmittelachse des Basistuchelements angeordnet ist. Der Abstand a beträgt vorzugsweise mindestens 2 %, weiter insbesondere mindestens 4 %, weiter insbesondere mindestens 6 %, weiter insbesondere höchstens 15 %, weiter insbesondere höchstens 12 %, weiter insbesondere höchstens 10 %, weiter insbesondere höchstens 8 % einer Distanz d in Querrichtung zwischen der Längsmittelachse des Basistuchelements und dem von der Längsmittelachse am weitesten entfernten Längsrandes des ersten und zweiten Längsrandes.

Mit einem Abstand, insbesondere mit diesen nominalen Abständen a des Lochausschnitts von der Längsmittelachse des Basistuchelements, wird in Bezug auf die im Wesentlichen symmetrische Anordnung des menschlichen Körpers vorteilhaft einem Versatz eines vorgesehenen Eingriffsbereichs links oder rechts von der Längsachse eines Patienten, insbesondere an den Gliedmaßen, Rechnung getragen.

Zu große Abstände des Lochausschnitts von der Längsmittelachse des Basistuchelements sind hingegen zu vermeiden, damit beidseits des Lochausschnitts genügend Basistuchmaterial zur Abdeckung zur Verfügung steht und zudem eine sichere Platzierung der Operationsabdeckung oberhalb und auf dem Patienten möglich ist.

Die Ausgestaltung des Lochausschnitts in seiner Form und/oder in seiner Erstreckung kann beliebig sein.

Insbesondere bevorzugt weist der Lochausschnitt eine polygonale, insbesondere eine rechteckige oder eine runde, insbesondere eine ovale Form auf. Polygonale oder runde Lochausschnitte sind im Herstellungsprozess leichter in das Basistuchelement einzubringen als ein Lochausschnitt mit einer eher komplizierten Kontur.

Bevorzugt erstreckt sich der Lochausschnitt weiter in der Längsrichtung als in der Querrichtung. Insbesondere weist der Lochausschnitt eine rechteckige Form mit kürzeren und längeren Seiten auf. Weiter insbesondere verlaufen die kürzeren Seiten der rechteckigen Form parallel zu dem ersten und zweiten Querrand und die längeren Seiten der rechteckigen Form parallel zu dem ersten und zweiten Längsrand.

Mit einer sich mehr in Längsrichtung erstreckenden, insbesondere rechteckigen Form, und insbesondere mit zu den Querrändern und den Längsrändern parallel verlaufenden Seiten kann der Lochausschnitt vorteilhaft leichter in das Basistuchelement eingebracht werden. Ein Lochausschnitt mit einer vergleichsweise größeren Längserstreckung ist vor allem bei Eingriffen an den Gliedmaßen vorteilhaft nutzbar.

Der Lochausschnitt weist vorzugsweise eine Fläche von mindestens 500 cm², insbesondere mindestens 1000 cm², weiter insbesondere mindestens 1500 cm², weiter insbesondere mindestens 2000 cm², und weiter insbesondere höchstens 5000 cm², weiter insbesondere höchstens 4500 cm², weiter insbesondere höchstens 4000 cm², weiter insbesondere höchstens 3500 cm², weiter insbesondere höchstens 3000 cm² auf.

Die kürzeren Seiten des Lochausschnittes weisen vorzugsweise eine Länge von 10 - 50 cm, insbesondere von 20 - 50 cm, weiter insbesondere von 30 - 50 cm auf, die längeren Seiten des Lochausschnitts weisen vorzugsweise eine Länge von 51 - 110 cm, insbesondere 51 - 100 cm, weiter insbesondere von 51 - 90 cm, weiter insbesondere von 51 - 80 cm auf.

Das Folienelement besteht vorzugsweise aus einem polymeren Material, insbesondere aus polymerem Material entnommen aus der Gruppe Polyethylen, Polypropylen, Polyurethan oder Mischungen davon. Das Folienelement weist vorzugsweise ein Flächengewicht von mindestens 10 g/m², insbesondere mindestens 15 g/m², weiter insbesondere mindestens 20 g/m², weiter insbesondere mindestens 25 g/m², weiter insbesondere von höchstens 50 g/m², weiter insbesondere höchstens 45 g/m², weiter insbesondere höchstens 40 g/m², weiter insbesondere höchstens 35 g/m² auf.

Vorzugsweise ist das Folienelement transparent. Damit ist bereits beim Anbringen des Folienelements auf den Patienten für den Anwender eine bessere Orientierung möglich. Zudem verschafft die Transparenz eine Einsehbarkeit direkt im Eingriffsbereich und in die am Eingriffsbereich direkt benachbarte Umgebung.

Für die klebende Ausbildung der Unterseite des Folienelements wird insbesondere eine Klebebeschichtung, vorzugsweise eine Haftkleberbeschichtung eingesetzt, insbesondere umfassend einen Polyacrylatkleber, insbesondere einen UV-vernetzten Polyacrylatkleber. Die Klebebeschichtung auf dem Folienelement weist vorzugsweise ein Flächengewicht von mindestens 10 g/m², insbesondere mindestens 15 g/m², weiter insbesondere mindestens 20 g/m², weiter insbesondere mindestens 25 g/m², weiter insbesondere von höchstens 60 g/m², weiter insbesondere höchstens 55 g/m², weiter insbesondere höchstens 50 g/m², weiter insbesondere höchstens 45 g/m², weiter insbesondere höchstens 40 g/m² auf.

Mit der klebenden Unterseite des Folienelements im Bereich des Lochausschnitts kann die Operationsabdeckung an den Patienten fixiert werden.

Ergänzend können vorzugsweise auf der Unterseite des Basistuchelements entlang des Lochausschnitts, vorzugweise vollumfänglich des Lochausschnitts zusätzlich Klebezonen vorgesehen sein, die eine noch bessere und damit sichere Fixierung der Operationsabdeckung an den Patienten ermöglichen.

In einer besonders bevorzugten Ausführung weist die wegwerfbare Operationsabdeckung ausschließlich im Bereich des Lochausschnitts eine klebende Unterseite auf. Die Reduktion des Klebebereichs ausschließlich auf den Bereich des Lochausschnitts trägt zum Komfort des Patienten bei, als dass bei einer möglichst geringen Kleberfläche auch weniger Belastung auf die Haut des Patienten ausgeübt wird und die Fläche des der Operation nachgeschalteten zumeist nicht immer schmerzfreien Ablösevorgangs der Operationsabdeckung vom Patienten minimiert wird. Zudem spart diese Ausführungsform Material und damit Kosten ein.

Die klebende Unterseite des Folienelements im Bereich des Lochausschnitts und/oder ergänzende zusätzliche Klebezonen auf der Unterseite des Basistuchelements sind vorzugsweise herstellerseitig mit einem Schutzmaterialelement, vorzugsweise einem sogenannten Releasestreifen, abgedeckt. Das Schutzmaterialelement kann Papier oder polymeres Material entnommen aus der Gruppe Polyethylen, Polypropylen, Polyurethan oder Mischungen umfassen oder daraus gebildet sein. Insbesondere kann das Schutzmaterialelement mit einer Beschichtung für Releaseverhalten, insbesondere mit Siloxanen ausgestattet sein.

Insbesondere sind auf der Operationsabdeckung bzw. dem Basistuchelement auch im Bereich der Querränder und/oder Längsränder keine Klebezonen vorgesehen. Als Operationsabdeckung, die oberhalb und auf dem Patienten abgelegt ist, insbesondere bei Operationen, die auch eine partielle Umlagerung von Körperteilen, insbesondere der unteren Gliedmaßen notwendig machen, wären weitere Kleberzonen eher nachteilig. Ablösevorgänge des Basistuchelements während der Operation könnten ansonsten die Gefahr eines Einreißens des Basistuchelements und damit einer Zerstörung der Sterilbarriere mit sich bringen.

Der absorbierende Materialabschnitt ist erfindungsgemäß unlösbar auf der Oberseite des Basistuchelements festgelegt. Die Befestigung des absorbierenden Materialabschnitts kann dabei vollflächig über seine gesamte Fläche oder auch nur bereichsweise, also nicht vollflächig, erfolgen.

In einer bevorzugten Ausführungsform kann vorgesehen sein, dass der absorbierende Materialabschnitt eine Fläche aufweist, die bereichsweise, insbesondere mit einem Befestigungsanteil von höchstens 80%, weiter insbesondere von höchstens 60%, weiter insbesondere von höchstens 40% bezogen auf die Fläche des absorbierenden Materialabschnitts auf der Oberseite des Basistuchelements befestigt ist.

Ein Befestigungsanteil von bevorzugt mindestens 10%, weiter insbesondere von mindestens 15%, weiter insbesondere von mindestens 20% bezogen auf die Fläche des absorbierenden Materialabschnitts ist vorteilhaft, um eine Unlösbarkeit des absorbierenden Materialabschnitts erreichen zu können.

Insbesondere ist der absorbierende Materialabschnitt, der Randbereiche aufweist, in den Randbereichen, insbesondere vollumfänglich, in ersten Fixierzonen auf der Oberseite des Basistuchelements befestigt.

Eine Befestigung des absorbierenden Materialabschnittes entlang von Randbereichen, insbesondere vollumfänglich, vermeidet vorteilhaft aufstehende Randbereiche des Materialabschnittes, welche beim Hantieren mit Instrumenten während des chirurgischen Eingriffs ein Hindernis darstellen können.

Die ersten Fixierzonen sind vorzugsweise im Wesentlichen bündig, mit höchstens einem geringen Abstand zu den Rändern des absorbierenden Materialabschnittes angeordnet. Hier sind insbesondere herstellungstechnisch bedingte geringe Abstände der ersten Fixierzonen vom Rand des absorbierenden Materialabschnittes, insbesondere Abstände bis zu 10 mm, noch im Sinne der Erfindung. Insbesondere beim Einsatz von in flüssigem oder fließfähigem Zustand aufgebrachten Fügemitteln, wie Kleber, wird damit ein Heraustreten des Fügemittels aus den zusammenzufügenden Lagen und somit ein unerwünschter Kleberüberstand vermieden.

Die ersten Fixierzonen weisen dabei vorzugsweise eine Breite b auf, die mindestens 1%, insbesondere mindestens 2%, weiter insbesondere höchstens 20%, weiter insbesondere höchstens 15%, weiter insbesondere höchstens 10% bezogen auf die längste Erstreckung in der Querrichtung EQ oder der Längsrichtung EL des absorbierenden Materialabschnitts beträgt. Das bedeutet, dass die Breite b auf EQ bezogen wird, wenn EQ größer ist als EL. Hingegen wird die Breite b auf EL bezogen, wenn EL größer als EQ ist.

Vorzugsweise weisen die ersten Fixierzonen eine Breite b von 10 - 100 mm, insbesondere eine Breite b von 10 - 80 mm, weiter insbesondere eine Breite b von 10 - 60 mm auf.

Die Fixierzonen müssen nicht vollflächig ausgefüllt sein, sondern es sind auch linien-, streifen- oder auch spiralförmige Ausgestaltungen denkbar. Bei der Bemessung der Breite der ersten Fixierzonen wird die Distanz zwischen den äußeren Rändern der Fixierzonen herangezogen.

In einer bevorzugten Ausführungsform können außerhalb der ersten Fixierzonen bereichsweise weitere Fixierzonen vorgesehen sind. Weitere Fixierzonen unterstützen vorteilhaft eine sichere und unlösbare Festlegung des absorbierenden Materialabschnittes auf dem Basistuchelement.

Die weiteren Fixierzonen können in jeglicher Form, insbesondere linien-, streifen- und/oder partiell flächenförmig gestaltet sein. Bei einem absorbierenden Materialabschnitt von insbesondere bevorzugter rechteckiger Form mit kürzeren und längeren Seiten können die weiteren Fixierzonen von bevorzugt linien- oder streifenförmiger Ausgestaltung mit ihrer Längserstreckung vorzugsweise parallel zu der längeren Seite oder vorzugsweise parallel zu der kürzeren Seite des absorbierenden Materialabschnittes angeordnet sein.

Die weiteren Fixierzonen sind vorzugsweise über die Fläche des absorbierenden Materialabschnitts homogen verteilt. Im Falle von externen Kräfteeinträge auf den absorbierenden Materialabschnitt führen diese bei einer gleichmäßigen und gleichförmigen Fixierung des absorbierenden Materialabschnitts eher weniger zu einer Gefahr des Ablösens des Materialabschnitts vom Basistuchelement.

In den ersten und/oder weiteren Fixierzonen können unterschiedliche Befestigungsmittel eingesetzt sein, wie beispielsweise Ultraschallverschweißungen, thermische Verschweißungen, doppelseitige Haftklebebänder und/oder ein Kleberauftrag, insbesondere ein linien-, streifen-, spiralförmiger und/oder vollflächiger Kleberauftrag.

In einer alternativen Ausführungsform kann bevorzugt vorgesehen sein, dass der absorbierende Materialabschnitt ausschließlich in den ersten Fixierzonen befestigt ist und ein zusammenhängender ungebundener Zwischenraum zwischen der Unterseite des Materialabschnitts und der Oberseite des Basistuchelements verbleibt.

Eine ausschließlich in den Randbereichen vorgenommene Fixierung des absorbierenden Materialabschnitts an das Basistuchelement erlaubt eine individuell und bedarfsangepasste Drapierung der verbliebenen ungebundenen Fläche des absorbierenden Materialabschnitts losgelöst vom Basistuchelement. Dies kann sich insbesondere bei der Anwendung des absorbierenden Materialabschnittes als eine Instrumentenablage als vorteilhaft erweisen, indem nestartige Materialanhäufungen drapiert werden können, welche eine Halterung von Instrumenten vorteilhaft unterstützen. Zudem stellt eine Fixierung des absorbierenden Materialabschnitts ausschließlich in ersten Fixierzonen in den Randbereichen des absorbierenden Materialabschnittes eine materialeinsparende und somit eine kostengünstige Variante dar.

Der absorbierende Materialabschnitt umfasst vorzugsweise Vliesmaterialien. Der absorbierende Materialabschnitt umfasst vorzugsweise einen ein- oder mehrlagigen Vliesstoff oder einen Vlies-Folien-Verbund, oder ist daraus gebildet.

Zur Unterstützung eines absorbierenden Verhaltens des Materialabschnittes werden vorzugsweise hydrophile Vliesmaterialien eingesetzt. Hierzu können beispielsweise bei Vliesmaterialen aus polymeren Fasern, insbesondere ausgewählt aus der Gruppe Polyethylen, Polypropylen, Polyamide, oder Kombinationen davon, die Fasern und/oder das Vliesmaterial mit einer hydrophilen Avivage behandelt sein. Vorzugsweise werden Vliesmaterialien umfassend absorbierende Fasern auf cellulosischer Basis, wie insbesondere Viskosefasern, eingesetzt.

Insbesondere vorteilhaft weist das im absorbierenden Materialabschnitt eingesetzte Vliesmaterial ein Flächengewicht von mindestens 40 g/m², weiter insbesondere mindestens 50 g/m², weiter insbesondere mindestens 60 g/m², weiter insbesondere von höchstens 150 g/m², weiter insbesondere höchstens 120 g/m², weiter insbesondere höchstens 100 g/m² , weiter insbesondere höchstens 80 g/m² auf.

Insbesondere Vliesmaterialien von höheren Flächengewichten tragen vorteilhaft zur Absorptionsfähigkeit bei. Zudem ist eine oftmals mit einem höheren Flächengewicht einhergehende Dicke und/oder Bauschigkeit des Vliesmaterials vorteilhaft für eine gut gepolsterte und damit sichere Ablage von Instrumenten auf dem absorbierenden Materialabschnitt.

Das Vliesmaterial kann dabei Spunbondvlieslagen und/oder Meltblownvlieslagen oder Stapelfasern umfassen.

Eine Abriebfestigkeit des Vliesmaterials, insbesondere bei Vliesmaterial aus Stapelfasern, wird vorteilhaft durch den Einsatz von Bindemitteln, sei es in Form von Bindefasern und/oder flüssigen Bindemitteln, erhalten.

Der absorbierende Materialabschnitt weist insbesondere bevorzugt auf der Oberseite bereichsweise Vlies-Fältelungen oder Vlies-Aufwerfungen auf.

Eine derartig nicht plane Oberseite bietet vorteilhaft eine größere Oberfläche zur Absorption von Flüssigkeit. Zudem kann in den zwischen den Vlies-Fältelungen oder Vlies-Aufwerfungen gebildeten Mulden leichter Flüssigkeit aufgefangen werden.

Während einer Operation können durchaus Situationen eintreten, die ein unvermitteltes Ablegen eines Instrumentes in direkter Nähe des Eingriffsfeldes erfordern. Solchen falls ist es vorteilhaft, wenn der auf der Oberseite des Basistuchelements angefügte absorbierende Materialabschnitt für eine solche Zwischenlagerung herangezogen werden kann, das heißt, dass der absorbierende Materialabschnitt auch die Funktion eines Instrumentenablagepads innehaben kann.

Insbesondere bevorzugt besteht der absorbierende Materialabschnitt aus einem Vlies-Folien-Laminat. Die Laminatbildung zwischen Vlies und Folie kann dabei mittels thermischer Bindung, einer Direktextrusion von Folienmaterial auf die Vlieslage oder durch Aufbringen/Einbringen von Fasermaterial auf noch nicht gefestigtes Folienmaterial erfolgen. Es ist auch die Verbindung des Vlies- und Folienmaterials mittels eines Klebematerials denkbar.

Die Ausführung des absorbierenden Materialabschnitts als ein Vlies-Folien-Laminat ist insbesondere vorteilhaft, wenn dem absorbierenden Materialabschnitt die Funktion einer kurzzeitigen Zwischenlagerung von Instrumenten, also eines Instrumentenablagepads, zugeteilt wird. Die Mehrlagigkeit, insbesondere auch der Einsatz einer Folie, unterstützt vorteilhaft die Stabilität des absorbierenden Materialabschnittes, so auch die Durchstichsicherheit, und damit wird allgemein die Sicherheit des absorbierenden Materialabschnitts erhöht.

Vlies-Fältelungen oder Vlies-Aufwerfungen können insbesondere bei der Ausführung des absorbierenden Materialabschnitts als Vlies-Folien-Laminat eingebracht werden, indem das Vlies-Folien-Laminat einer thermischen Behandlung ausgesetzt wird. Eine thermische Behandlung kann auch im Zuge eines Sterilisationsschrittes erfolgen. Durch eine thermische Behandlung von miteinander verbundenen Lagen von unterschiedlich ausgeprägten thermischen Schrumpfverhalten können unterschiedliche Materialerhebungen resultieren. Bei einem Folienmaterial mit einem vergleichsweise stärker ausgeprägtem Schrumpfverhalten als beim Vliesmaterial, führt dies im Folienmaterial zu einer stärkeren Verkürzung als im Vliesmaterial, wodurch Vlies-Fältelungen oder Vlies-Aufwerfungen entstehen.

Für die Bereitstellung einer Rutschhemmung, insbesondere mit Blick auf die Funktion des absorbierenden Materialabschnitts als ein Instrumentenablagepad, ist es insbesondere bevorzugt, wenn der absorbierende Materialabschnitt auf der Oberseite einen dynamischen Reibungskoeffizienten gemessen nach ASTM D 1894-01 von mindestens 0,15, insbesondere von mindestens 0,2, weiter insbesondere von mindestens 0,25, weiter insbesondere von mindestens 0,3 aufweist.

Insbesondere bei einer Oberseite des absorbierenden Materialabschnitts, die aus einer Materiallage mit einer Maschinenrichtung und einer Querrichtung gebildet ist, wie insbesondere bei einem Vliesmaterial, weist die Oberseite den vorgenannten dynamischen Gleitreibungskoeffizient bei Messung in Maschinenrichtung und/oder in Querrichtung der Materiallage auf.

### Test zur Ermittlung des dynamischen Gleitreibungskoeffizienten:

Vorliegend soll das Rutschverhalten von der Oberseite des absorbierenden Materialabschnitts der erfindungsgemäßen wegwerfbaren Operationsabdeckung ermittelt werden. Hierbei wird die Oberseite des absorbierenden Materialabschnitts gegenüber einer standardisierten Oberfläche gezogen. Die hierbei auftretende Gleitreibungskraft A soll gemessen und hieraus dann der dynamische Gleitreibungskoeffizient ermittelt werden. Die Prüfmethode ist angelehnt an die ASTM D 1894-01, zur Bestimmung des Reibungsverhaltens von Kunststoff-Folien.

Die Prüfkörper müssen mindestens 2 Stunden im Normklima bei 23° C ± 2°C und 50% ± 2% Luftfeuchtigkeit konditioniert sein. Die Proben dürfen nicht geknickt, gefaltet oder zerkratzt sein; sonstige Veränderungen und Verunreinigungen sind zu vermeiden. Dasselbe gilt für die Prüfplatte aus Stahl. Das Prüfverfahren ist ebenso unter Normbedingungen (23°C ± 2°C, 50 % ± 2%) durchzuführen.

Aus der wegwerfbaren Operationsabdeckung wird im Bereich des absorbierenden Materialabschnitts oder aus einer entsprechenden Rollenware wird ein Probenkörper einer Abmessung von 65 x 200 mm ausgestanzt und faltenfrei an einem Reibklotz befestigt. Bei der Rollenware handelt es sich aber um exakt dasjenige Material, aus dem der absorbierende Materialabschnitt der erfindungsmäßen wegwerfbaren Operationsabdeckung gebildet ist. Die Probenkörper werden im Falle einer Oberseiten bildenden Materiallage mit einer Maschinen- und einer Querrichtung dabei sowohl in der Maschinenrichtung (MD) als in der Querrichtung (CD) der die Oberseite des absorbierenden Materialabschnitts bildenden Lage ausgestanzt.

Der Reibklotz weist eine Grundfläche von 63 mm x 63 mm Kantenlänge, also eine Kontaktgrundfläche von 40 cm² und eine Masse von 200 g ± 5g auf. Er wird über einen Faden (ohne Eigendehnung) am Kraftaufnehmer einer Zugprüfmaschine nach DIN EN ISO 7500-01:2004-11 befestigt. Eine solche Zugprüfmaschine ist das Prüfgerät Zwick Roell Typ Z010 von der Firma Zwick GmbH&Co.KG, 89079 Ulm, Deutschland.

Das Zusatzgerät bestehend aus dem Probentisch und Reibklotz nach DIN EN ISO 8295:2004-10 wird ebenfalls von der Firma Zwick angeboten. Der Reibklotz mit dem Probenkörper wird auf einen definierten Werkstoff, eine glatt polierte Stahlplatte (DIN EN 1939: 2003-12) vorsichtig aufgelegt. 15 Sekunden nach dem Auflegen des Reibklotzes wird der Versuch gestartet. Die Prüfgeschwindigkeit beträgt 150 mm/min, sowohl für den eigentlichen Messweg von 130 mm, als auch für den Vor- und Nachmessweg von jeweils 10 mm. Für die Ermittlung des dynamischen Gleitreibungskoeffizienten µ wird nur der Kraftverlauf des Messwegs von 130 mm herangezogen. Der Test wird für wenigstens fünf Prüfkörper durchgeführt. Bei Oberseiten bildenden Materiallagen mit einer Differenzierung von Maschinenrichtung und Querrichtung werden hierzu jeweils fünf Prüfkörper in Maschinenrichtung und Querrichtung vermessen. Es wird ein Mittelwert x und die Standardabweichung s auf zwei Nachkommastellen gerundet angegeben. Der dynamische Gleitreibungskoeffizient ergibt sich aus dem Quotienten der so ermittelten Gleitreibungskraft A ausgedrückt in Gramm (g) durch die durch den Reibklotz ausgeübte Kraft von 200 g.

Der absorbierende Materialabschnitt weist vorzugsweise ein Flächengewicht von 20 - 180g/m², insbesondere von 40 - 150 g/m², weiter insbesondere von 50 - 120 g/m², weiter insbesondere von 50 - 100 g/m² auf.

In einer bevorzugten Ausführung erstreckt sich der absorbierende Materialabschnitt weiter in der Längsrichtung als in der Querrichtung, insbesondere weist der absorbierende Materialabschnitt eine rechteckige Form mit kürzeren und längeren Seiten auf. Insbesondere bevorzugt verlaufen die kürzeren Seiten der rechteckigen Form parallel zum ersten und zweiten Querrand des Basistuchelements und die längeren Seiten verlaufen parallel zum ersten und zweiten Längsrand des Basistuchelements.

Der absorbierende Materialabschnitt weist vorzugsweise eine Fläche von mindestens 1000 cm², insbesondere mindestens 2000 cm², weiter insbesondere mindestens 3000 cm², weiter insbesondere mindestens 4000 cm², und weiter insbesondere höchstens 8000 cm², weiter insbesondere höchstens 7000 cm², weiter insbesondere höchstens 6000 cm², weiter insbesondere höchstens 5000 cm² auf.

Die längeren Seiten des absorbierenden Materialabschnitts weisen vorzugsweise eine Länge von 51 - 150 cm, insbesondere von 51 - 140 cm, weiter insbesondere von 51 - 130 cm, weiter insbesondere von 51 -1 20 cm auf, die kürzeren Seiten des absorbierenden Materialabschnitts weisen vorzugsweise eine Länge von 20 - 50 cm, insbesondere von 25 - 50 cm, weiter insbesondere von 30 - 50 cm auf.

Vorzugsweise weist der absorbierende Materialabschnitt eine im Vergleich zum Lochausschnitt größere Erstreckung in Längsrichtung auf.

Eine größere Längserstreckung des absorbierenden Materialabschnitts ermöglicht vorteilhaft auch den neben dem Chirurgen stehenden und assistierenden Mitarbeitern eine bessere Erreichbarkeit des absorbierenden Materialabschnitts und damit auch Zugriffs-und Ablagemöglichkeit von Instrumenten.

Der Flüssigkeitsauffangbeutel kann jegliche Form aufweisen. Der Flüssigkeitsauffangbeutel ist vorzugsweise aus einem polymeren Flachbahnmaterial, insbesondere aus einer Folie gebildet. Insbesondere ist der Flüssigkeitsauffangbeutel transparent ausgebildet, um damit vorteilhaft das Volumen der sich ansammelnden Flüssigkeit beobachten zu können.

Für die Festlegung des Flüssigkeitsauffangbeutels auf der Oberseite des Basistuchelements können unterschiedliche Befestigungsmittel eingesetzt sein, wie beispielsweise Ultraschallverschweißungen, thermische Verschweißungen, doppelseitige Haftklebebänder und/oder ein Kleberauftrag.

Erfindungsgemäß ist die Beutelöffnung des Flüssigkeitsauffangbeutels dem Lochausschnitt zugewandt. Vorzugsweise erstreckt sich die Beutelöffnung dabei parallel zur Längsrichtung des Basistuchelements und/oder parallel zu den Seiten des Lochausschnittes.

Vorzugsweise ist der Flüssigkeitsauffangbeutel unter Beibehalt einer funktionalen Beutelöffnung zumindest entlang eines Abschnittes der Beutelöffnung auf der Oberseite des Basistuchelements festgelegt. Vorzugsweise ist der Flüssigkeitsauffangbeutel ausschließlich entlang von Abschnitten der Beutelöffnung festgelegt, so dass im Anwendungsfall bei der Befüllung mit Flüssigkeit vorteilhaft keine großflächigen Kräfteeinträge auf die Fläche des Basistuchelements ausübt werden.

Das Basistuchelement weist vorzugsweise ein Flächengewicht von mindestens 20 g/m², insbesondere mindestens 30 g/m², weiter insbesondere mindestens 40 g/m², weiter insbesondere von höchstens 120 g/m², weiter insbesondere höchstens 100 g/m², weiter insbesondere höchstens 80 g/m², weiter insbesondere höchstens 60 g/m² auf.

In einer bevorzugten Ausführung der wegwerfbaren Operationsabdeckung kann am Lochausschnitt auf der Oberseite des Basistuchelements ein Vliesmaterial vorgesehen sein, wobei das Vliesmaterial insbesondere in der Querrichtung zwischen dem Lochausschnitt und dem Flüssigkeitsauffangbeutel und/oder in der Querrichtung zwischen dem Lochausschnitt und dem absorbierenden Materialabschnitt angeordnet ist. Insbesondere ist am Lochausschnitt ein Rahmen aus Vliesmaterial vorgesehen.

Das direkt am Lochausschnitt vorgesehene Vliesmaterial, insbesondere ein Rahmen aus Vliesmaterial, kann vorteilhaft als eine Orientierungshilfe über die Erstreckung des, eines insbesondere mit einem transparenten Folienelement überfangenden Lochausschnitts gegenüber dem den Lochausschnitt umgebenden Randbereich aus transparentem Flachbahnmaterial dienen. Zusätzlich können damit, insbesondere bei einem hydrophilen Vliesmaterial, auch Absorptionsflächen für aus dem Eingriffsbereich austretende Flüssigkeiten bereitgestellt werden. Vorzugsweise umfasst das direkt am Lochausschnitt vorgesehene Vliesmaterial ein- oder mehrlagige Spinnvlies- und/oder Meltblownlagen.

Die Ausstattung des Basistuchelements zumindest in einem Randbereich des Lochausschnitts mit einem transparenten Flachbahnmaterial gewährt dem Arzt und dem medizinischen Personal vorteilhaft eine Einsehbarkeit auf den Patienten in direkter Nachbarschaft zum Eingriffsbereich.

In einer bevorzugten Ausführung erstreckt sich der Randbereich des transparenten Flachbahnmaterials beidseits der Quermittelachse in der Längsrichtung mindestens über ein Sechstel, insbesondere mindestens über ein Viertel der Längserstreckung des Basistuchelements. Und weiter insbesondere erstreckt sich der Randbereich des transparenten Flachbahnmaterials in der Querrichtung über die gesamte Quererstreckung des Basistuchelements.

Ein sich derart weiter in Längsrichtung erstreckender Randbereich aus transparentem Flachbahnmaterial ist vor allem von Vorteil, wenn während der Operation Funktionsprüfungen am Patienten vorgenommen werden müssen. Vor allem bei Operationen im Bereich der unteren Gliedmaßen besteht der Bedarf, dass die Stellung der unteren Gliedmaßen während der Operation geändert, angeglichen und damit auch beobachtet und überprüft werden muss.

Insbesondere bevorzugt ist das Basistuchelement aus einem transparenten Flachbahnmaterial, insbesondere einer Folie, gebildet.

Bei einem vollständig aus einem transparenten, insbesondere einer Folie, gebildeten Basistuchelement können die Vorteile über die Möglichkeit der Einsehbarkeit auf den Patienten mit einem einfachen und sicherem und damit auch kostengünstigen Herstellungsprozess verbunden werden.

Das transparente Flachbahnmaterial ist insbesondere eine Folie, insbesondere eine Folie umfassend oder gebildet aus Polyolefinen.

Der Einsatz einer Folie hat auch den Vorteil, dass der Eigenschaft des Folienmaterials inhärent, nicht die Problematik des Lintings, also der Ablösung von kleinen Faserteilchen, vorliegt, was im Operationsumfeld unbedingt vermieden werden muss. Deshalb ist es insbesondere vorteilhaft in den Randbereichen des Lochausschnitts, also um den Eingriffsbereich, als transparentes Flachbahnmaterial eine Folie einzusetzen.

Das transparente Flachbahnmaterial, insbesondere die Folie, weist vorzugsweise eine Dicke von mindestens 20 µm, insbesondere mindestens 40 µm, weiter insbesondere mindestens 60 µm, weiter insbesondere höchstens 120 µm, weiter insbesondere höchstens 100 µm, weiter insbesondere höchstens 80 µm auf. Ein Flachbahnmaterial, insbesondere eine Folie mit solchen Werte an Dicke, trägt vorteilhaft zur Stabilität, wie u.a. auch Reißfestigkeit, Durchstichfestigkeit, bei, was insbesondere in den Randbereichen um den Lochausschnitt, also dem eigentlichen Eingriffsbereich während der Operation positiv ist, aber dennoch auch eine Drapierfähigkeit an den Patienten ermöglicht.

Die Dickenmessung wird mittels eines mechanischen Dickenmessgeräts unter Anwendung eines spezifischen Messdrucks von 2 g/cm² auf einer Tasterfläche von 25 cm² durchgeführt.

Im Falle, dass das Basistuchelement nicht aus einem transparenten Flachbahnmaterial, insbesondere einer Folie, gebildet ist, werden für das Basistuchelement außerhalb des Randbereichs des Lochausschnitts aus transparenten Flachbahnmaterials bevorzugt Vliesmaterialien und/ oder Vlies-Folien-Verbundmaterialien eingesetzt. Als Vliesmaterialien können vorzugsweise ein ein- oder mehrlagiger Vliesstoff, insbesondere ein Laminat aus einer oder mehreren Spinnvlies- und/oder Meltblownvlieslagen und/oder als Vlies-Folien-Verbundmaterialien, insbesondere Vlies-Folien-Verbundmaterialien mit einem Vliesstoff mit ein oder mehreren Lagen aus Spinnvlies und/oder Meltblown eingesetzt werden. Spinnvlies- und/oder Meltblownvlieslagen umfassen vorzugsweise polymere Materialien, insbesondere ausgewählt aus der Gruppe Polyethylen, Polypropylen oder Mischungen, insbesondere ein hydrophilisiertes Polypropylen.

In einer weiteren bevorzugten Ausführungsform der wegwerfbaren Operationsabdeckung sind auf der Oberseite des Basistuchelements, insbesondere in Längsrichtung zwischen dem Lochausschnitt und dem ersten und/oder zweiten Querrand bandförmige Instrumenten-, Kabel- und/oder Schlauch-Halterungsvorrichtungen und/oder taschenförmige Instrumenten-, Kabel- und/oder Schlauch-Halterungsvorrichtungen vorgesehen.

Mit der Ausstattung mit solchen Halterungsvorrichtungen stehen dem medizinischen Personal während der Operation bedarfsgerecht weitere Hilfskomponenten zur Verfügung, um ein Abrutschen von Instrumenten, Kabeln oder Schläuchen unterhalb der Operationsebene zu vermeiden. Mit einer Anordnung der Halterungsvorrichtungen insbesondere beidseits in Längsrichtung zum Lochausschnitt wird der universellen Einsetzbarkeit der Operationsabdeckung vorteilhaft Rechnung getragen.

Bei der wegwerfbaren Operationsabdeckung handelt es sich vorzugsweise um eine untere Gliedmaßen-Operationsabdeckung, insbesondere um eine untere Rumpf- und/oder Becken-Operationsabdeckung, eine Hüft-Operationsabdeckung, eine Oberschenkel-Operationsabdeckung, eine Knie-Operationsabdeckung, eine Unterschenkel-Operationsabdeckung und/oder eine Fuß-Operationsabdeckung.

Das Basistuchelement weist vorzugsweise eine Fläche von 6 - 15 m², insbesondere 6 - 12 m², weiter insbesondere 6-10 m² auf.

Das Basistuchelement erstreckt sich vorzugsweise weiter in der Längsrichtung als in der Querrichtung, insbesondere weist das Basistuchelement eine rechteckige Form mit kürzeren und längeren Seiten auf.

Die längeren Seiten des Basistuchelements weisen vorzugsweise eine Länge von 300 - 700 cm, insbesondere von 300 - 600 cm, weiter insbesondere von 300 - 500 cm auf, die kürzeren Seiten des Basistuchelements weisen vorzugsweise eine Länge von 200 - 300 cm, insbesondere von 200 - 250 cm auf.

Mit solchen Maßen des Basistuchelements ist eine ausreichende Abdeckung des Patienten möglich.

Weitere Merkmale, Vorteile und Einzelheiten der Erfindung ergeben sich aus den beigefügten Patentansprüchen und aus der zeichnerischen Darstellung und der nachfolgenden Beschreibung bevorzugter Ausführungsformen der Erfindung.

Die Erfindung soll im Folgenden anhand von Figuren näher erläutert werden, dabei zeigen
- Figur 1:: schematisch eine erfindungsmäße wegwerfbare Operationsabdeckung in einer Draufsicht,
- Figur 2a:: schematisch einen Ausschnitt einer erfindungsgemäßen wegwerfbaren Operationsabdeckung mit Draufsicht auf einen absorbierenden Materialabschnitt,
- Figur 2b:: schematisch eine Schnittansicht I - I von Figur 2a,
- Figur 3a:: schematisch einen Ausschnitt einer weiteren erfindungsgemäßen wegwerfbaren Operationsabdeckung mit Draufsicht auf einen absorbierenden Materialabschnitt,
- Figur 3b:: schematisch eine Schnittansicht II - II von Figur 3a.

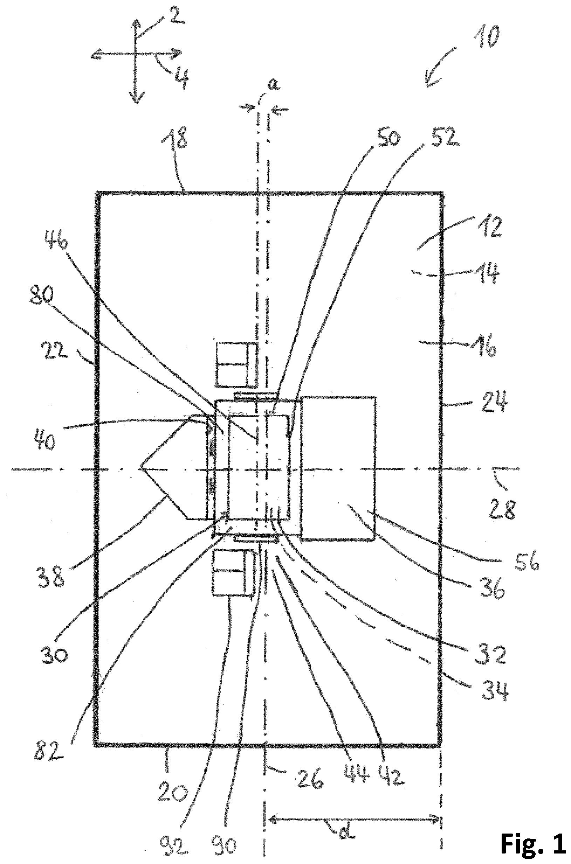

Figur 1 zeigt schematisch eine Draufsicht einer bevorzugten wegwerfbaren Operationsabdeckung 10 mit einer Längsrichtung 2 und mit einer Querrichtung 4 und mit einer Oberseite 12 und einer Unterseite 14 umfassend ein Basistuchelement 16 mit einem einzigen Lochausschnitt 30. Zusammen mit der weiteren Ausstattung mit einem Flüssigkeitsauffangbeutel 38 und mit einem absorbierenden Materialabschnitt 36, welche beide auf der Oberseite 12 des Basistuchelements 16 angebracht sind, ist die Anordnung zueinander wesentlich, um damit eine universal einsetzbare wegwerfbare Operationsabdeckung 10 bereitzustellen. Der Lochausschnitt 30, der Flüssigkeitsauffangbeutel 38 und der absorbierende Materialabschnitt 36 sind jeweils auf der Quermittelachse 28 des Basistuchelements 16 angeordnet, dabei ist der Flüssigkeitssauffangbeutel 38 mit einer dem Lochausschnitt zugewandten Beutelöffnung 40 neben dem Lochausschnitt 30 in Richtung zum ersten oder zweiten Längsrand 22, 24 des Basistuchelements 16 und der absorbierende Materialabschnitt 36 neben dem Lochausschnitt 30 in Richtung zum jeweils anderen ersten oder zweiten Längsrand 22, 24 angeordnet. In einer besonders vorteilhaften Ausführung sind der Lochausschnitt 30, der Flüssigkeitsauffangbeutel 38 und der absorbierende Materialabschnitt 36 in der Längsrichtung 2 zentriert auf der Quermittelachse 28 angeordnet.

Durch die Anordnung der wesentlichen Komponenten Lochausschnitt, Flüssigkeitsauffangbeutel und absorbierendem Materialabschnitt auf der Quermittelachse 28, kann die wegwerfbare Operationsabdeckung je nach Präferenzen oder Arbeitsweise des Chirurgen, sei es linkshändig oder rechtshändig, oder linksseitig oder rechtsseitig vom Patienten oder auch je nach Position des vorzunehmenden Eingriffsbereiches, um 180° gedreht werden, so dass der Flüssigkeitsauffangbeutel lateral zum Patienten angeordnet ist und der absorbierende Materialabschnitt medial zur Längsachse des Patienten, und damit auf dem Patienten zum Liegen kommen kann.

Für den Einsatz der wegwerfbaren Operationsabdeckung vor allem für Operationen an den Gliedmaßen, welche am menschlichen Körper weitgehend symmetrisch vorliegen, so kann die erfindungsgemäße Operationsabdeckung vorteilhaft sowohl für Operationen an den links von der menschlichen Längsachse als an den rechts davon angeordneten Gliedmaßen angewendet werden.

In dieser Anordnung von Lochausschnitt 30, Flüssigkeitsauffangbeutel 38 und absorbierendem Materialabschnitt 36 entlang der Quermittelachse 28 ist es insbesondere vorteilhaft, wenn der Lochausschnitt 30 auf der Längsmittelachse 26 des Basistuchelements 16 positioniert ist.

"Auf der Quermittelachse 28" oder "auf der Längsmittelachse 26" wird verstanden, dass die Komponenten mit ihrer flächenhaften Erstreckung die jeweilige Quermittelachse oder Längsmittelachse zumindest tangieren.

Wie in der Figur 1 ersichtlich, so ist der Lochausschnitt 30 nicht in der Querrichtung 4 zentriert auf der Längsmittelachse 26 angeordnet, sondern nimmt davon eine leicht versetzte Position ein, so dass der Abstand zum ersten Längsrand 22 größer oder kleiner als der Abstand zum zweiten Längsrand 24 ist. Der Versatz des Lochausschnitts von der Längsmittelachse 26 des Basistuchelements 16 lasst sich vorzugsweise als Abstand a der dem Lochausschnitt zueignen Lochausschnittlängsmittelachse 46 zur Längsmittelachse 26 des Basistuchelements 16 beschreiben. Der Abstand a beträgt vorzugsweise mindestens 2% und insbesondere höchstens 15% einer Distanz d in Querrichtung 4 zwischen der Längsmittelachse 26 des Basistuchelements 16 und dem weitesten entfernten Längsrand des ersten und zweiten Längsrandes 22, 24. In einer bevorzugten Alternative kann der Lochausschnitt 30 durchaus in der Querrichtung zentriert auf der Längsmittelachse 26 angeordnet sein.

Das Basistuchelement 16 weist zumindest im Randbereich 42 des Lochausschnitts 30 ein transparentes Flachbahnmaterial 44, insbesondere eine Folie, auf. Um eine noch bessere Einsehbarkeit auf den Patienten zu haben, erstreckt sich das transparente Flachbahnmaterial 44, bzw. dieser Randbereich 42, beidseits der Quermittelachse 28 in der Längsrichtung 2 über mindestens ein Sechstel, vorzugsweise über mindestens ein Viertel der Längserstreckung des Basistuchelements 16. Vorzugsweise erstreckt sich dieser Randbereich mit transparentem Flachbahnmaterial über die gesamte Querrichtung 4. Vor allem bei Operationen mit der Notwendigkeit von Umlagerungen und/oder Drehungen von Körperteilen, insbesondere der unteren Gliedmaßen des Patienten, ist ein großer transparenter Bereich vorteilhaft. Bei einem vorzugsweise vollständig aus transparentem Flachbahnmaterial, insbesondere transparenter Folie gebildeten Basistuchelement gestaltet sich zudem der Herstellungsprozess einfacher.

Der Lochausschnitt 30 mit einer vorzugsweisen rechteckigen Form mit kürzeren Seiten 50, die vorzugsweise parallel zu dem ersten und zweiten Querrand 18, 20 und mit längeren Seiten 52, die vorzugsweise parallel zu dem ersten und zweiten Längsrand 22, 24 verlaufen, insbesondere mit einer bevorzugten Fläche von 2000-3000 cm², ist von einem Folienelement 32 überfangen. Die Unterseite 14 des Folienelements 32 ist zumindest im Bereich des Lochausschnitts 30 klebend ausgebildet, und weist insbesondere eine Klebebeschichtung 34 auf.

Die wegwerfbare Operationsabdeckung 10 ist vorzugsweise mit bandförmigen Instrumenten-, Kabel- und/oder Schlauch-Halterungsvorrichtungen 90 und mit taschenförmigen Instrumenten-, Kabel- und/oder Schlauch-Halterungsvorrichtungen 92 ausgestattet. Dabei sind diese Halterungsvorrichtungen 90, 92 vorzugsweise in der Längsrichtung 2 zwischen dem Lochausschnitt 30 und dem ersten und/oder zweiten Querrand 18, 20 des Basistuchelements 16 angeordnet.

Am Lochausschnitt 30 auf der Oberseite des Basistuchelements 16 ist vorzugsweise ein Vliesmaterial 80 angeordnet, insbesondere in der Querrichtung 4 zwischen Lochausschnitt 30 und Flüssigkeitsauffangbeutel 38 und zwischen Lochausschnitt 30 und dem absorbierenden Materialabschnitt 36. Insbesondere ist ein Rahmen 82 aus Vliesmaterial 80 vorgesehen. Das Vliesmaterial 80 kann neben einer gewissen Flüssigkeitsaufnahme auch als Orientierungshilfe und Markierung der Kontur des mit einem insbesondere transparenten Folienelement 32 überfangenen Lochausschnitts 30 gegenüber dem Randbereich 42 des Lochausschnitts 30 aus einem transparenten Flachbahnmaterial 44, insbesondere auch einer Folie, hilfreich sein.

Der absorbierende Materialabschnitt 36 erstreckt sich vorzugsweise weiter in der Längsrichtung als in der Querrichtung, insbesondere weist der absorbierende Materialabschnitt eine rechteckige Form mit kürzeren und längeren Seiten auf. Die kürzeren Seiten der rechteckigen Form verlaufen bevorzugt parallel zum ersten und zweiten Querrand (18, 20) des Basistuchelements 16 und die längeren Seiten verlaufen parallel zum ersten und zweiten Längsrand (22,24) des Basistuchelements 16.

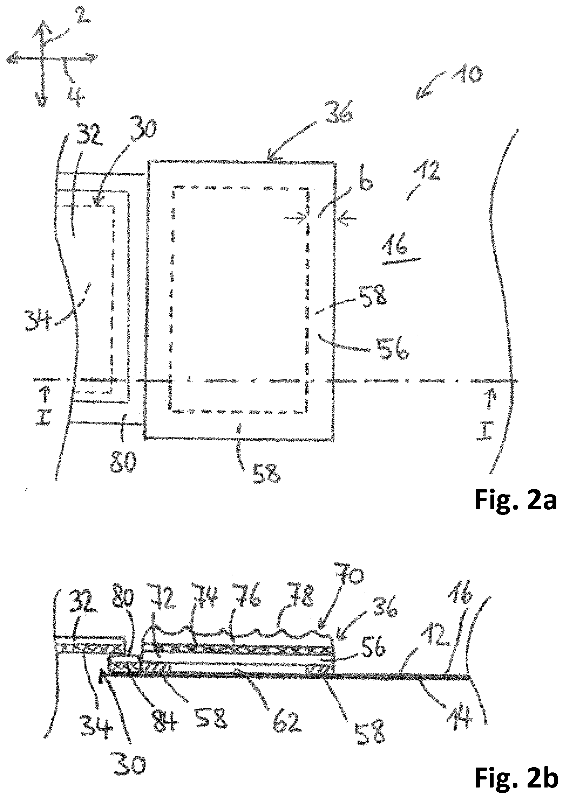

Der absorbierende Materialabschnitt 36 ist auf der Oberseite 12 des Basistuchelements 16 unlösbar festgelegt. Die Fixierung des Materialabschnitts wird dabei vorzugsweise in den Randbereichen 56 des Materialabschnitts 36, insbesondere vollumfänglich, in ersten Fixierzonen 58 vorgenommen, wie schematisch in Figur 2a als ein Ausschnitt einer erfindungsgemäßen wegwerfbaren Operationsabdeckung 10 mit Draufsicht auf einen absorbierenden Materialabschnitt 36 dargestellt ist. Vorzugsweise ist die Fixierung des absorbierenden Materialabschnitts auf diese ersten Fixierzonen 58 beschränkt. Das heißt, es sind keine weiteren Fixierzonen vorgesehen, so dass zwischen der Unterseite 14 des absorbierenden Materialabschnitts 36 und der Oberseite 12 des Basistuchelements 16 ein zusammenhängender ungebundener Zwischenraum 62 verbleibt, wie in Figur 2b als eine Schnittansicht I-I der Figur 2a dargestellt ist. Mit dieser ausschließlich randbereichsständigen Fixierung kann die verbleibende ungebundene Fläche des absorbierenden Materialabschnitts nach Bedarf drapiert werden.

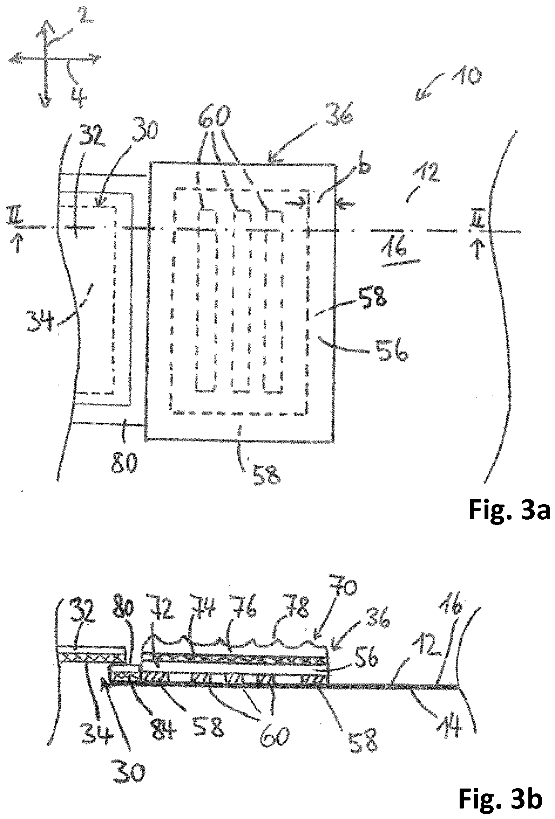

Es sind jedoch auch Ausführungen denkbar, in denen der absorbierende Materialabschnitt 36 bereichsweise durch weitere Fixierzonen 60 festgelegt wird, wobei diese weiteren Fixierzonen 60 in jeglicher Form vorgesehen sein können, wie beispielsweise streifenförmig, wie schematisch in der Figur 3a und in der Figur 3b als Schnittansicht II- II von Figur 3a dargestellt ist.

Die Breite b der ersten Fixierzonen 58 bemisst sich vorzugsweise zwischen 2 - 20% der längsten Erstreckung. der Querrichtung EQ oder der Längsrichtung EL des absorbierenden Materialabschnitts. Das bedeutet, dass die Breite b auf EQ bezogen wird, wenn EQ größer ist als EL. Hingegen wird die Breite b auf EL bezogen, wenn EL größer als EQ ist. Vorzugsweise beträgt die Breite b 10 - 100 mm.

Alternativ kann eine vollflächige Fixierung des absorbierenden Materialabschnitts auf die Oberseite des Basistuchelements vorgesehen sein.

Der absorbierende Materialabschnitt 36 umfasst vorzugsweise Vliesmaterialien, die auf der Oberseite 12 bereichsweise Vlies-Fältelungen oder Vlies-Aufwerfungen 78 aufweisen, wie schematisch in den Figuren 2b und 3b dargestellt ist. Die Vlies-Fältelungen oder Vlies-Aufwerfungen 78 schaffen vorteilhaft eine vergrößerte Oberfläche zur Absorption und Aufnahme von Flüssigkeiten. Zudem können die damit einhergehenden Unebenheiten positiv für eine Rutschhemmung für darauf zwischengelagerte Instrumente beitragen.

Um für solche durchaus situationsbedingten kurzfristigen Zwischenlagerungen von Instrumenten auch eine Stabilität und Sicherheit gegenüber Durchstich positiv beitragen zu können, ist der absorbierende Materialabschnitt 36 bevorzugt ein Vlies-Folien-Laminat 70 bei der das Vlies 76 die Oberseite 12 darstellt. Die Laminatbildung zwischen Vlies 76 und Folienmaterial 72 kann insbesondere mittels eines Klebematerials 74 erfolgen.

Das den Lochausschnitt 30 überfangende Folienelement 32 ist auf der Unterseite 14 insbesondere mit einer Kleberbeschichtung 34 belegt. Damit ist das Folienelement zumindest im Bereich des Lochausschnitts klebend ausgebildet und kann im Anwendungszustand an den Patienten fixiert werden. Vorzugsweise ist in den über den Lochausschnitt 30 hinauserstreckenden Bereichen des Folienelements 32 ebenso die Kleberbeschichtung 34 vorgesehen, mit der das Folienelement 32 auf der Oberseite 12 des Basistuchelements 16 festgelegt werden kann. Im Falle eines am Lochausschnitt 30 auf der Oberseite 12 des Basistuchelements vorgesehenen und mittels eines Klebers 84 fixierten Vliesmaterials 80, wie schematisch in den Figuren 2b und 3b dargestellt, kann das Folienelement 30 mit seiner Klebebeschichtung 34 auch das Vliesmaterial 80 teilweise überlappen und darauf festgelegt sein.

Die wegwerfbare Operationsabdeckung findet ihren Einsatz insbesondere als eine untere Gliedmaßen-Operationsabdeckung, insbesondere als eine untere Rumpf- und/oder Becken-Operationsabdeckung insbesondere als eine Hüft-Operationsabdeckung, eine Oberschenkel-Operationsabdeckung, eine Knie-Operationsabdeckung, eine Unterschenkel-Operationsabdeckung, eine Fuß-Operationsabdeckung.

## Patentansprüche

1. Wegwerfbare Operationsabdeckung (10) mit einer Längsrichtung (2) und mit einer Querrichtung (4) und mit einer Oberseite (12) und einer Unterseite (14) umfassend ein flüssigkeitsundurchlässiges Basistuchelement (16) mit einem ersten und einem zweiten Querrand (18, 20), und mit einem ersten und einem zweiten Längsrand (22, 24), und mit einer Längsmittelachse (26) und mit einer Quermittelachse (28), und mit einem einzigen innerhalb des Basistuchelements (16) angeordneten Lochausschnitt (30), wobei der Lochausschnitt von einem Folienelement (32) überfangen ist und das Folienelement (32) zumindest im Bereich des Lochausschnitts (30) auf der Unterseite (14) klebend ausgebildet ist, insbesondere eine Klebebeschichtung (34) aufweist, wobei ein Flüssigkeitsauffangbeutel (38) mit einer dem Lochausschnitt (30) zugewandten Beutelöffnung (40) auf der Oberseite (14) des Basistuchelements (16) festgelegt ist, **dadurch gekennzeichnet, dass** das Basistuchelement (16) zumindest in einem Randbereich (42) des Lochausschnitts (30) ein transparentes Flachbahnmaterial (44) aufweist, wobei der Lochausschnitt (30) auf der Quermittelachse (28) angeordnet ist, wobei der Flüssigkeitsauffangbeutel (38) auf der Quermittelachse (28) in der Querrichtung (4) zwischen dem Lochausschnitt (30) und dem ersten oder dem zweiten Längsrand (22, 24) angeordnet ist und wobei ein absorbierender Materialabschnitt (36) auf der Oberseite (14) des Basistuchelements (16) unlösbar festgelegt ist, wobei der absorbierende Materialabschnitt (56) auf der Quermittelachse (28) in der Querrichtung (4) zwischen dem Lochausschnitt (30) und dem jeweils anderen ersten oder zweiten Längsrand (22, 24) angeordnet ist.

2. Wegwerfbare Operationsabdeckung (10) nach Anspruch 1, **dadurch gekennzeichnet, dass** der Lochausschnitt (30), der absorbierende Materialabschnitt (36) und der Flüssigkeitsauffangbeutel (38) in der Längsrichtung (2) zentriert auf der Quermittelachse (28) angeordnet sind.

3. Wegwerfbare Operationsabdeckung (10) nach Anspruch 1 oder 2, **dadurch gekennzeichnet, dass** der Lochausschnitt (30) auf der Längsmittelachse (26) angeordnet ist.

4. Wegwerfbare Operationsabdeckung (10) nach Anspruch 1, 2 oder 3, **dadurch gekennzeichnet, dass** der Lochausschnitt (30) in der Querrichtung (4) zentriert auf der Längsmittelachse (26) angeordnet ist.

5. Wegwerfbare Operationsabdeckung (10) nach Anspruch 1, 2 oder 3, **dadurch gekennzeichnet, dass** der Lochausschnitt (30) in der Querrichtung (4) nicht zentriert auf der Längsmittelachse (26) angeordnet ist, so dass ein Abstand des Lochausschnitts (30) zu dem ersten Längsrand (22) kleiner oder größer ist als ein Abstand des Lochausschnitts (3) zu dem zweiten Längsrand (24).

6. Wegwerfbare Operationsabdeckung (10) nach Anspruch 5, **dadurch gekennzeichnet, dass** der Lochausschnitt (30) eine Lochausschnittmittellängsachse (46) aufweist und, dass die Lochausschnittmittellängsachse (46) in einem Abstand a versetzt zur Längsmittelachse (26) des Basistuchelements angeordnet ist, insbesondere dass der Abstand a mindestens 2 %, weiter insbesondere mindestens 4 %, weiter insbesondere mindestens 6 %, weiter insbesondere höchstens 15 %, weiter insbesondere höchstens 12 %, weiter insbesondere höchstens 10 %, weiter insbesondere höchstens 8 % einer Distanz d in Querrichtung (4) zwischen der Längsmittelachse (26) des Basistuchelements und dem von der Längsmittelachse (26) am weitesten entfernten Längsrandes des ersten und zweiten Längsrandes (22, 24) beträgt.

7. Wegwerfbare Operationsabdeckung (10) nach einem der vorangehenden Ansprüche, **dadurch gekennzeichnet, dass** der Lochausschnitt (30) sich weiter in der Längsrichtung (2) als in der Querrichtung (4) erstreckt, insbesondere eine rechteckige Form mit kürzeren und längeren Seiten (50, 52) aufweist, und weiter insbesondere die kürzeren Seiten (50) der rechteckigen Form parallel zu dem ersten und zweiten Querrand (18, 20) und die längeren Seiten (52) der rechteckigen Form parallel zu dem ersten und zweiten Längsrand (22, 24) verlaufen.

8. Wegwerfbare Operationsabdeckung (10) nach einem der vorangehenden Ansprüche, **dadurch gekennzeichnet, dass** der absorbierende Materialabschnitt (36) eine Fläche aufweist, die bereichsweise, insbesondere mit einem Befestigungsanteil von höchstens 80%, insbesondere höchstens 60%, weiter insbesondere höchstens 40% bezogen auf die Fläche des absorbierenden Materialabschnitts (36), und weiter insbesondere mit einem Befestigungsanteil von mindestens 10%, insbesondere mindestens 15%, weiter insbesondere mindestens 20% bezogen auf die Fläche des absorbierenden Materialabschnitts auf der Oberseite (12) des Basistuchelements (16) befestigt ist.

9. Wegwerfbare Operationsabdeckung (10) nach einem der vorangehenden Ansprüche, **dadurch gekennzeichnet, dass** der absorbierende Materialabschnitt (36) Randbereiche (56) aufweist und der absorbierende Materialabschnitt (36) in den Randbereichen (56), insbesondere vollumfänglich, in ersten Fixierzonen (58) auf der Oberseite (12) des Basistuchelements (16) befestigt ist.

10. Wegwerfbare Operationsabdeckung (10) nach Anspruch 9, **dadurch gekennzeichnet, dass** die ersten Fixierzonen (58) eine Breite b aufweisen, die mindestens 1%, insbesondere mindestens 2 %, weiter insbesondere höchstens 20%, weiter insbesondere höchstens 15%, weiter insbesondere höchstens 10% bezogen auf die längste Erstreckung in Querrichtung (4) oder in Längsrichtung (2) des absorbierenden Materialabschnitts (36) beträgt, weiter insbesondere dass die ersten Fixierzonen (58) eine Breite b von 10 - 100 mm, insbesondere eine Breite b von 10 - 80 mm, weiter insbesondere eine Breite b von 10 - 60 mm aufweisen.

11. Wegwerfbare Operationsabdeckung (10) nach Anspruch 9 oder 10, **dadurch gekennzeichnet, dass** außerhalb der ersten Fixierzonen (58) bereichsweise weitere Fixierzonen (60) vorgesehen sind oder dass der absorbierende Materialabschnitt (36) ausschließlich in den ersten Fixierzonen (58) befestigt ist und ein zusammenhängender ungebundener Zwischenraum (62) zwischen der Unterseite (14) des Materialabschnitts (36) und der Oberseite (12) des Basistuchelements (16) verbleibt.

12. Wegwerfbare Operationsabdeckung (10) nach einem der vorgenannten Ansprüche, **dadurch gekennzeichnet, dass** der absorbierende Materialabschnitt (36) Vliesmaterialien umfasst, insbesondere einen ein- oder mehrlagigen Vliesstoff oder einen Vlies-Folien-Verbund (70) umfasst oder daraus gebildet ist.

13. Wegwerfbare Operationsabdeckung (10) nach einem der vorgenannten Ansprüche, **dadurch gekennzeichnet, dass** der absorbierende Materialabschnitt (36) auf der Oberseite (12) bereichsweise Vlies-Fältelungen oder Vlies-Aufwerfungen (78) aufweist.

14. Wegwerfbare Operationsabdeckung (10) nach einem der vorgenannten Ansprüche, **dadurch gekennzeichnet, dass** der absorbierende Materialabschnitt (36) auf der Oberseite (14) einen Reibungskoeffizienten gemessen nach ASTM D 1894-01 von mindestens 0, 15, insbesondere von mindestens 0,2, weiter insbesondere von mindestens 0,25, weiter insbesondere von mindestens 0,3 aufweist.

15. Wegwerfbare Operationsabdeckung (10) nach einem der vorgenannten Ansprüche, **dadurch gekennzeichnet, dass** der absorbierende Materialabschnitt (36) ein Flächengewicht von 20 - 180 g/m², insbesondere von 40 - 150 g/m², weiter insbesondere von 50 - 120 g/m², weiter insbesondere von 50 - 100 g/m² aufweist.

16. Wegwerfbare Operationsabdeckung (10) nach einem der vorgenannten Ansprüche, **dadurch gekennzeichnet, dass** das Basistuchelement (16) ein Flächengewicht von mindestens 20 g/m², insbesondere mindestens 30 g/m², weiter insbesondere mindestens 40 g/m², weiter insbesondere von höchstens 120 g/m², weiter insbesondere höchstens 100 g/m², weiter insbesondere höchstens 80 g/m², weiter insbesondere höchstens 60 g/m² aufweist.

17. Wegwerfbare Operationsabdeckung (10) nach einem der vorgenannten Ansprüche, **dadurch gekennzeichnet, dass** am Lochausschnitt (30) auf der Oberseite (12) des Basistuchelements (16) ein Vliesmaterial (80) vorgesehen ist, wobei das Vliesmaterial (80) insbesondere in der Querrichtung (4) zwischen dem Lochausschnitt (30) und dem Flüssigkeitsauffangbeutel (38) und/oder in der Querrichtung (4) zwischen dem Lochausschnitt (30) und dem absorbierenden Materialabschnitt (36) angeordnet ist, wobei insbesondere am Lochausschnitt (30) ein Rahmen (82) aus Vliesmaterial (80) vorgesehen ist.

18. Wegwerfbare Operationsabdeckung (10) nach einem der vorgenannten Ansprüche, **dadurch gekennzeichnet, dass** der Randbereich (42) des transparenten Flachbahnmaterials (44) sich beidseits der Quermittelachse (28) in der Längsrichtung (2) mindestens über ein Sechstel, insbesondere mindestens über ein Viertel der Längserstreckung des Basistuchelements (16) erstreckt, und insbesondere, dass der Randbereich (42) des transparenten Flachbahnmaterials (44) sich in der Querrichtung (4) über die gesamte Quererstreckung des Basistuchelements (16) erstreckt.

19. Wegwerfbare Operationsabdeckung (10) nach einem der vorgenannten Ansprüche, **dadurch gekennzeichnet, dass** das Basistuchelement (16) aus einem transparenten Flachbahnmaterial (44), insbesondere einer Folie gebildet ist.

20. Wegwerfbare Operationsabdeckung (10) nach einem der vorgenannten Ansprüche, **dadurch gekennzeichnet, dass** auf der Oberseite (14) des Basistuchelements (16), insbesondere in Längsrichtung (2) zwischen dem Lochausschnitt (30) und dem ersten und/oder zweiten Querrand (18, 20) bandförmige Instrumenten-, Kabel- und/oder Schlauch-Halterungsvorrichtungen (90) und/oder taschenförmige Instrumenten-, Kabel- und/oder Schlauch-Halterungsvorrichtungen (92) vorgesehen sind.

21. Wegwerfbare Operationsabdeckung (10) nach einem der vorgenannten Ansprüche, **dadurch gekennzeichnet, dass** die wegwerfbare Operationsabdeckung (10) eine untere Gliedmaßen-Operationsabdeckung, insbesondere eine untere Rumpf -und/oder Becken-Operationsabdeckung. insbesondere eine Hüft- Operationsabdeckung, eine Oberschenkel-Operationsabdeckung, eine Knie-Operationsabdeckung, eine Unterschenkel-Operationsabdeckung und/oder eine Fuß-Operationsabdeckung ist.

## Claims

1. Disposable surgical drape (10) with a longitudinal direction (2) and a transverse direction (4) and with an upper face (12) and a lower face (14), comprising a liquid-impermeable base cloth element (16) with a first and a second transverse edge (18, 20), a first and a second longitudinal edge (22, 24), a longitudinal central axis (26) and a transverse central axis (28), and a single cutout hole (30) arranged within the base cloth element (16), wherein the cutout hole is covered by a film element (32), and the film element (32) is designed to be adhesive on the lower face (14), at least in the region of the cutout hole (30), and in particular has an adhesive coating (34), wherein a liquid-collecting bag (38) with a bag opening (40) directed towards the cutout hole (30) is fastened to the upper face (14) of the base cloth element (16), **characterized in that** the base cloth element (16) has a transparent flat web material (44) at least in an edge region (42) of the cutout hole (30), wherein the cutout hole (30) is arranged on the transverse central axis (28), wherein the liquid-collecting bag (38) is arranged on the transverse central axis (28) between the cutout hole (30) and the first or second longitudinal edge (22, 24) in the transverse direction (4), and wherein an absorbent material portion (36) is fastened permanently to the upper face (14) of the base cloth element (16), wherein the absorbent material portion (56) is arranged on the transverse central axis (28) between the cutout hole (30) and the respective other first or second longitudinal edge (22, 24) in the transverse direction (4).

2. Disposable surgical drape (10) according to Claim 1, **characterized in that** the cutout hole (30), the absorbent material portion (36) and the liquid-collecting bag (38) are arranged in the longitudinal direction (2) in a manner centred on the transverse central axis (28).

3. Disposable surgical drape (10) according to Claim 1 or 2, **characterized in that** the cutout hole (30) is arranged on the longitudinal central axis (26).

4. Disposable surgical drape (10) according to Claim 1, 2 or 3, **characterized in that** the cutout hole (30) is arranged in the transverse direction (4) in a manner centred on the longitudinal central axis (26) .

5. Disposable surgical drape (10) according to Claim 1, 2 or 3, **characterized in that** the cutout hole (30) is arranged in the transverse direction (4) in a manner not centred on the longitudinal central axis (26), such that a distance from the cutout hole (30) to the first longitudinal edge (22) is less than or greater than a distance from the cutout hole (3) to the second longitudinal edge (24).

6. Disposable surgical drape (10) according to Claim 5, **characterized in that** the cutout hole (30) has a cutout hole central longitudinal axis (46), and **in that** the cutout hole central longitudinal axis (46) is arranged offset at a distance a from the longitudinal central axis (26) of the base cloth element, in particular **in that** the distance a measures at least 2%, more particularly at least 4%, more particularly at least 6%, more particularly at most 15%, more particularly at most 12%, more particularly at most 10%, more particularly at most 8% of a distance d in the transverse direction (4) between the longitudinal central axis (26) of the base cloth element and the longitudinal edge, farthest from the longitudinal central axis (26), of the first and second longitudinal edges (23, 24).

7. Disposable surgical drape (10) according to one of the preceding claims, **characterized in that** the cutout hole (30) extends farther in the longitudinal direction (2) than in the transverse direction (4) and in particular has a rectangular shape with shorter and longer sides (50, 52), and more particularly the shorter sides (50) of the rectangular shape extend parallel to the first and second transverse edges (18, 20), and the longer sides (52) of the rectangular shape extend parallel to the first and second longitudinal edges (22, 24).

8. Disposable surgical drape (10) according to one of the preceding claims, **characterized in that** the absorbent material portion (36) has a surface area which is fastened to the upper face (12) of the base cloth element (16) regionally, in particular with a fastening proportion of at most 80%, in particular at most 60%, more particularly at most 40% relative to the surface area of the absorbent material portion (36), and more particularly with a fastening proportion of at least 10%, in particular at least 15%, more particularly at least 20% relative to the surface area of the absorbent material portion.

9. Disposable surgical drape (10) according to one of the preceding claims, **characterized in that** the absorbent material portion (36) has edge regions (56), and the absorbent material portion (36) is fastened to the upper face (12) of the base cloth element (16) in the edge regions (56), in particular about the whole circumference, in first fixing zones (58) .

10. Disposable surgical drape (10) according to Claim 9, **characterized in that** the first fixing zones (58) have a width b which measures at least 1%, in particular at least 2%, more particularly at most 20%, more particularly at most 15%, more particularly at most 10% relative to the longest extent in the transverse direction (4) or in the longitudinal direction (2) of the absorbent material portion (36), more particularly **in that** the first fixing zones (58) have a width b of 10-100 mm, in particular a width b of 10-80 mm, more particularly a width b of 10-60 mm.

11. Disposable surgical drape (10) according to Claim 9 or 10, **characterized in that**, outside the first fixing zones (58), further fixing zones (60) are provided in some regions, or **in that** the absorbent material portion (36) is fastened exclusively in the first fixing zones (58), and a continuous unbonded interspace (62) remains between the lower face (14) of the material portion (36) and the upper face (12) of the base cloth element (16).

12. Disposable surgical drape (10) according to one of the preceding claims, **characterized in that** the absorbent material portion (36) comprises nonwoven materials, in particular comprises or is formed from a single-ply or multi-ply nonwoven or a nonwoven film composite (70).

13. Disposable surgical drape (10) according to one of the preceding claims, **characterized in that** the absorbent material portion (36) has nonwoven pleats or nonwoven folds (78) in some regions on the upper face (12).

14. Disposable surgical drape (10) according to one of the preceding claims, **characterized in that** the absorbent material portion (36) on the upper face (14) has a coefficient of friction, measured according to ASTM D 1894-01, of at least 0.15, in particular of at least 0.2, more particularly of at least 0.25, more particularly of at least 0.3.

15. Disposable surgical drape (10) according to one of the preceding claims, **characterized in that** the absorbent material portion (36) has a basis weight of 20-180 g/m², in particular of 40-150 g/m², more particularly of 50-120 g/m², more particularly of 50-100 g/m².

16. Disposable surgical drape (10) according to one of the preceding claims, **characterized in that** the base cloth element (16) has a basis weight of at least 20 g/m², in particular at least 30 g/m², more particularly at least 40 g/m², more particularly at most 120 g/m², more particularly at most 100 g/m², more particularly at most 80 g/m², more particularly at most 60 g/m².

17. Disposable surgical drape (10) according to one of the preceding claims, **characterized in that** a nonwoven material (80) is provided at the cutout hole (30) on the upper face (12) of the base cloth element (16), wherein the nonwoven material (80) is arranged in particular in the transverse direction (4) between the cutout hole (30) and the liquid-collecting bag (38) and/or in the transverse direction (4) between the cutout hole (30) and the absorbent material portion (36), wherein in particular a frame (82) of nonwoven material (80) is provided at the cutout hole (30).

18. Disposable surgical drape (10) according to one of the preceding claims, **characterized in that** the edge region (42) of the transparent flat web material (44) extends on both sides of the transverse central axis (28) in the longitudinal direction (2) over at least one sixth, in particular over at least one quarter of the longitudinal extent of the base cloth element (16), and in particular **in that** the edge region (42) of the transparent flat web material (44) extends in the transverse direction (4) over the entire transverse extent of the base cloth element (16).

19. Disposable surgical drape (10) according to one of the preceding claims, **characterized in that** the base cloth element (16) is formed from a transparent flat web material (44), in particular a film.

20. Disposable surgical drape (10) according to one of the preceding claims, **characterized in that** band-shaped instrument, cable and/or hose holders (90) and/or pocket-shaped instrument, cable and/or hose holders (92) are provided on the upper face (14) of the base cloth element (16), in particular in the longitudinal direction (2) between the cutout hole (30) and the first and/or second transverse edge (18, 20).

21. Disposable surgical drape (10) according to one of the preceding claims, **characterized in that** the disposable surgical drape (10) is a surgical drape for the lower extremities, in particular a surgical drape for the lower trunk and/or pelvis, in particular a surgical drape for the hips, a surgical drape for the thighs, a surgical drape for the knees, a surgical drape for the lower legs and/or a surgical drape for the feet.

## Revendications

1. Couverture chirurgicale jetable (10) ayant une direction longitudinale (2) et ayant une direction transversale (4) et ayant un côté supérieur (12) et un côté inférieur (14) comprenant un élément toile de base imperméable aux liquides (16) ayant un premier et un deuxième bord transversal (18, 20), et ayant un premier et un deuxième bord longitudinal (22, 24), et ayant un axe médian longitudinal (26) et ayant un axe médian transversal (28), et ayant une découpe de trou individuelle (30) agencée au sein de l'élément toile de base (16), la découpe de trou étant recouverte par un élément feuille (32) et l'élément feuille (32) étant configuré sous forme adhésive au moins dans la région de la découpe de trou (30) sur le côté inférieur (14), notamment comprenant un revêtement adhésif (34), une poche de collecte de liquide (38) ayant une ouverture de poche (40) tournée vers la découpe de trou (30) étant fixée sur le côté supérieur (14) de l'élément toile de base (16), **caractérisée en ce que** l'élément toile de base (16) comprend, au moins dans une région de bord (42) de la découpe de trou (30), un matériau en bande plate transparent (44), la découpe de trou (30) étant agencée sur l'axe médian transversal (28), la poche de collecte de liquide (38) étant agencée sur l'axe médian transversal (28) dans la direction transversale (4) entre la découpe de trou (30) et le premier ou le deuxième bord longitudinal (22, 24), et une section de matériau absorbant (36) étant fixé de manière non amovible sur le côté supérieur (14) de l'élément toile de base (16), la section de matériau absorbant (56) étant agencée sur l'axe médian transversal (28) dans la direction transversale (4) entre la découpe de trou (30) et respectivement l'autre premier ou deuxième bord longitudinal (22, 24).

2. Couverture chirurgicale jetable (10) selon la revendication 1, **caractérisée en ce que** la découpe de trou (30), la section de matériau absorbant (36) et la poche de collecte de liquide (38) sont agencées centrées sur l'axe médian transversal (28) dans la direction longitudinale (2).

3. Couverture chirurgicale jetable (10) selon la revendication 1 ou 2, **caractérisée en ce que** la découpe de trou (30) est agencée sur l'axe médian longitudinal (26) .

4. Couverture chirurgicale jetable (10) selon la revendication 1, 2 ou 3, **caractérisée en ce que** la découpe de trou (30) est agencée centrée sur l'axe médian longitudinal (26) dans la direction transversale (4) .

5. Couverture chirurgicale jetable (10) selon la revendication 1, 2 ou 3, **caractérisée en ce que** la découpe de trou (30) est agencée non centrée sur l'axe médian longitudinal (26) dans la direction transversale (4), de telle sorte qu'un écart de la découpe de trou (30) au premier bord longitudinal (22) soit inférieur ou supérieur à un écart de la découpe de trou (3) au deuxième bord longitudinal (24).

6. Couverture chirurgicale jetable (10) selon la revendication 5, **caractérisée en ce que** la découpe de trou (30) présente un axe longitudinal médian de découpe de trou (46), et **en ce que** l'axe longitudinal médian de découpe de trou (46) est agencé à un écart a en décalage par rapport à l'axe médian longitudinal (26) de l'élément toile de base, notamment **en ce que** l'écart a est d'au moins 2 %, plus particulièrement d'au moins 4 %, plus particulièrement d'au moins 6 %, plus particulièrement d'au plus 15 %, plus particulièrement d'au plus 12 %, plus particulièrement d'au plus 10 %, plus particulièrement d'au plus 8 % d'une distance d dans la direction transversale (4) entre l'axe médian longitudinal (26) de l'élément toile de base et le bord longitudinal le plus éloigné de l'axe médian longitudinal (26) parmi le premier et le deuxième bord longitudinal (22, 24).

7. Couverture chirurgicale jetable (10) selon l'une quelconque des revendications précédentes, **caractérisée en ce que** la découpe de trou (30) s'étend plus loin dans la direction longitudinale (2) que dans la direction transversale (4), notamment présente une forme rectangulaire ayant des côtés plus courts et plus longs (50, 52), et plus particulièrement les côtés plus courts (50) de la forme rectangulaire sont parallèles au premier et deuxième bord transversal (18, 20) et les côtés plus longs (52) de la forme rectangulaire sont parallèles au premier et deuxième bord longitudinal (22, 24).

8. Couverture chirurgicale jetable (10) selon l'une quelconque des revendications précédentes, **caractérisée en ce que** la section de matériau absorbant (36) présente une surface qui est fixée en régions, notamment avec une proportion de fixation d'au plus 80 %, particulièrement d'au plus 60 %, plus particulièrement d'au plus 40 %, par rapport à la surface de la section de matériau absorbant (36), et plus particulièrement avec une proportion de fixation d'au moins 10 %, particulièrement d'au moins 15 %, plus particulièrement d'au moins 20 %, par rapport à la surface de la section de matériau absorbant, sur le côté supérieur (12) de l'élément toile de base (16).

9. Couverture chirurgicale jetable (10) selon l'une quelconque des revendications précédentes, **caractérisée en ce que** la section de matériau absorbant (36) comprend des régions de bord (56) et la section de matériau absorbant (36) est fixée dans les régions de bord (56), particulièrement en intégralité, dans des premières zones de fixation (58) sur le côté supérieur (12) de l'élément toile de base (16).

10. Couverture chirurgicale jetable (10) selon la revendication 9, **caractérisée en ce que** les premières zones de fixation (58) présentent une largeur b, qui est d'au moins 1 %, particulièrement d'au moins 2 %, plus particulièrement d'au plus 20 %, plus particulièrement d'au plus 15 %, plus particulièrement d'au plus 10 %, par rapport à l'étendue la plus longue dans la direction transversale (4) ou dans la direction longitudinale (2) de la section de matériau absorbant (36), plus particulièrement **en ce que** les premières zones de fixation (58) présentent une largeur b de 10 à 100 mm, particulièrement une largeur b de 10 à 80 mm, plus particulièrement une largeur b de 10 à 60 mm.

11. Couverture chirurgicale jetable (10) selon la revendication 9 ou 10, **caractérisée en ce qu'**en dehors des premières zones de fixation (58), des zones de fixation supplémentaires (60) sont prévues en régions, ou **en ce que** la section de matériau absorbant (36) est fixée exclusivement dans les premières zones de fixation (58) et un espace intermédiaire non relié cohérent (62) reste entre le côté inférieur (14) de la section de matériau (36) et le côté supérieur (12) de l'élément toile de base (16).

12. Couverture chirurgicale jetable (10) selon l'une quelconque des revendications précédentes, **caractérisée en ce que** la section de matériau absorbant (36) comporte des matériaux non tissés, particulièrement comporte un non-tissé mono- ou multicouche ou un composite de feuilles non tissées (70) ou en est formée.

13. Couverture chirurgicale jetable (10) selon l'une quelconque des revendications précédentes, **caractérisée en ce que** la section de matériau absorbant (36) comprend en régions sur le côté supérieur (12) des plissements de non-tissé ou des élévations de non-tissé (78) .

14. Couverture chirurgicale jetable (10) selon l'une quelconque des revendications précédentes, **caractérisée en ce que** la section de matériau absorbant (36) présente sur le côté supérieur (14) un coefficient de frottement mesuré selon ASTM D 1894-01 d'au moins 0, 15, particulièrement d'au moins 0,2, plus particulièrement d'au moins 0,25, plus particulièrement d'au moins 0,3.

15. Couverture chirurgicale jetable (10) selon l'une quelconque des revendications précédentes, **caractérisée en ce que** la section de matériau absorbant (36) présente un poids surfacique de 20 à 180 g/m², particulièrement de 40 à 150 g/m², plus particulièrement de 50 à 120 g/m², plus particulièrement de 50 à 100 g/m².

16. Couverture chirurgicale jetable (10) selon l'une quelconque des revendications précédentes, **caractérisée en ce que** l'élément toile de base (16) présente un poids surfacique d'au moins 20 g/m², particulièrement d'au moins 30 g/m², plus particulièrement d'au moins 40 g/m², plus particulièrement d'au plus 120 g/m², plus particulièrement d'au plus 100 g/m², plus particulièrement d'au plus 80 g/m², plus particulièrement d'au plus 60 g/m².

17. Couverture chirurgicale jetable (10) selon l'une quelconque des revendications précédentes, **caractérisée en ce qu'**un matériau non tissé (80) est prévu au niveau de la découpe de trou (30) sur le côté supérieur (12) de l'élément toile de base (16), le matériau non tissé (80) étant agencé particulièrement dans la direction transversale (4) entre la découpe de trou (30) et la poche de collecte de liquide (38) et/ou dans la direction transversale (4) entre la découpe de trou (30) et la section de matériau absorbant (36), particulièrement un cadre (82) en matériau non tissé (80) étant prévu au niveau de la découpe de trou (30).

18. Couverture chirurgicale jetable (10) selon l'une quelconque des revendications précédentes, **caractérisée en ce que** la région de bord (42) du matériau en bande plate transparent (44) s'étend des deux côtés de l'axe médian transversal (28) dans la direction longitudinale (2) au moins sur un sixième, particulièrement au moins sur un quart de l'étendue longitudinale de l'élément toile de base (16), et particulièrement, **en ce que** la région de bord (42) du matériau en bande plate transparent (44) s'étend dans la direction transversale (4) sur l'étendue transversale totale de l'élément toile de base (16).

19. Couverture chirurgicale jetable (10) selon l'une quelconque des revendications précédentes, **caractérisée en ce que** l'élément toile de base (16) est formé d'un matériau en bande plate transparent (44), notamment d'une feuille.

20. Couverture chirurgicale jetable (10) selon l'une quelconque des revendications précédentes, **caractérisée en ce que** des dispositifs de maintien d'instruments, de câbles et/ou de tuyaux en forme de bande (90) et/ou des dispositifs de maintien d'instruments, de câbles et/ou de tuyaux en forme de sac (92) sont prévus sur le côté supérieur (14) de l'élément toile de base (16), particulièrement dans la direction longitudinale (2) entre la découpe de trou (30) et le premier et/ou deuxième bord transversal (18, 20).

21. Couverture chirurgicale jetable (10) selon l'une quelconque des revendications précédentes, **caractérisée en ce que** la couverture chirurgicale jetable (10) est une couverture chirurgicale pour membres inférieurs, particulièrement une couverture chirurgicale pour le tronc inférieur et/ou le bassin, particulièrement une couverture chirurgicale pour la hanche, une couverture chirurgicale pour la cuisse, une couverture chirurgicale pour le genou, une couverture chirurgicale pour la jambe et/ou une couverture chirurgicale pour le pied.
